# EUROPEAN PATENT APPLICATION

(11) **EP 2 679 495 A2**
(43) Date of publication of application: **01.01.2014**
(21) Application number: 13173938.5
(22) Date of filing: 27.06.2013
(51) Int. Cl.: B64D 11/00, B64D 13/00, B64D 13/06

(54) **A passenger service unit comprising a ventilation nozzle and a reading light**

(30) Priority: 28.06.2012 US 201261665486 P
(71) Applicant: INTERTECHNIQUE, 78373 Plaisir Cedex (FR)
(72) Inventor: Rittner, Wolfgang, 23623 Ahrensbök (DE); Boomgaarden, Günter, 23684 Scharbeutz (DE); Hollm, Marco, 25548 Rosdorf (DE); Meckes, Rüdiger, 23919 Berkenthin (DE); Weinmann, Hasso, 23564 Lübeck (DE)
(74) Representative: Eisenführ Speiser

(57) **Abstract**

The invention relates to a passenger service unit 1 comprising a reading light 3 and a ventilation nozzle 2. The ventilation nozzle is pivotably mounted within a frame 4 wherein the frame is fixed to the passenger service unit. A ring shape-element 12 is arranged circumferentially around the ventilation nozzle and incorporates a plurality of light means 3 providing a light beam to the passenger. The light means are adjusted by an adjustment means 11 which effects a relative movement of the light means to the ring-shaped element. The ring-shaped element is immovable fixed to the frame.

## Description

The invention relates to a passenger service unit in an airplane comprising a reading light and a ventilation nozzle.

Generally, passenger aircrafts have a cabin in which the passengers are seated. For every seat or at least for every group of seats one or more passenger service units (PSU) are installed in a ceiling compartment of the cabin. Usually, every PSU comprises a reading light and a ventilation nozzle being separated from each other. The ventilation nozzle provides an air flow for the passenger. In order to adjust the direction of the airflow, the ventilation nozzle may be mounted within a spherical housing in a first area of the PSU. The passenger is able to swivel the ventilation nozzle in said spherical housing by grasping the outer surface of the nozzle.

The reading light emits a light beam which is directed towards the passenger's seat. The reading light is usually fixed to the PSU, so that the passenger is not able to change the direction of the light beam. The direction of the light beam is usually determined when the reading light is assembled to the PSU.

Since one PSU is usually provided for each of the passenger seats, the number of reading lights and ventilation nozzles can be very high, in particular in large aircrafts. Therefore, the separate arrangement of both items causes weight and demands space. Even if the weight and space reduction of the items is small, it may decrease the fuel consumption and costs while increasing the comfort and the operator convenience.

From US 2008/0112155A1 it is known to integrate the reading light in the personal air outlet. A ring comprising a plurality of light means is mounted inside a spherical bearing for a pivotal adjustment. By this, the direction of the light means may be adjusted by pivoting the ring. Furthermore, an air nozzle is mounted inside a spherical bearing of said ring for a pivotal adjustment allowing to adjust the direction of the air flow. The passenger may adjust the air flow by grasping the outer surface of the air nozzle. The adjustment of the light beam effects a change of the direction of the air flow from the air nozzle. An independent adjustment of the light means only is not possible.

From US4142227 and CA1111010 it is known to combine an orientable light and ventilating fixture. An electrical lamp is adapted to a socket, wherein the socket is mounted to a ball housing therein. The ball housing is adjustably mounted to a support wherein a bezel element is connected with the ball housing. By adjusting the bezel element the direction of the air flow, which flows through the ball housing and around the electrical lamp, is varied. Because of the rigid connection between the electrical lamp and the ball housing, an adjustment of the bezel element always causes an adjustment of the electrical lamp. Therefore, the direction of the light beam and the direction of the air flow are dependent from each other. An independent adjustment of the light beam and the air flow is not possible.

Accordingly, it is a major object of the present invention to improve the arrangement of the ventilation nozzle and the reading light in a PSU for reducing costs and increasing the comfort for the passenger.

The problem is solved by a PSU comprising a ventilation nozzle and a reading light, wherein the reading light surrounds the ventilation nozzle. A plurality of light means is mounted to a ring-shaped element which is fixed to the frame. The light means are movably in relation to the ring-shaped element by an adjustment means which is coupled to the light means. When the ventilation nozzle is adjusted by grasping its outer surface, the direction of the light beam of the light means does not change. The other way around, when the light means are adjusted by moving the adjustment means, the direction of the air flow of the ventilation nozzle does not change. That implies that the adjustment of the ventilation nozzle and the light means are independent from each other.

In a first aspect of the invention the ventilation nozzle is mounted pivotably within a frame, wherein the frame is connectable to the PSU. The frame is preferably connected to the PSU by screwing, gluing or clamping. The outlet of the ventilation nozzle is faced towards the passenger. The passenger can direct the air flow of the ventilation nozzle by pivoting the air nozzle within the frame. Therefore, he grasps the outer surface of the ventilation nozzle with his hand and pushes or swivels the nozzle into a desired direction.

A plurality of light means circumscribes the ventilation nozzle. Each of the light means emits a light beam which is directed in a projecting direction. The plurality of light means is mounted to a ring-shaped element which is preferably clamped or glued immovable to the frame. The ring-shaped element has a circular shape having an outer and inner circular edge, wherein the distance between the inner and outer circular edge is sufficient to incorporate a light means in between. Instead of moving the ring for adjusting the light beam as known from prior art, the passenger moves the light means in relation to the ring-shaped element by the adjustment means. Therefore, the adjustment means is coupled to the light means, so that a movement of the adjustment means effects a relative movement of the light means to the ring-shaped element.

According to a first preferred embodiment the ring-shaped element is integral in the frame or immovable mounted, preferably screwed, glued or clamped, as a separate or integral part to the frame.

According to a second preferred embodiment the ring-shaped element has a plurality of recesses to incorporate the plurality of light means. The ring-shaped element has an inner and outer circular edge having the plurality of recesses in-between. The number of recesses in the ring-shaped element may correspond to the number of light means. Preferably the recess is a bore. The bore has e.g. a truncated cone, wherein the circular area having the smaller diameter is an opening facing towards the passenger. Furthermore, the smaller diameter of the circular area of the truncated cone may be smaller than the diameter of the preferably spherical light means. Therefore, an outer spherical surface of the light means may contact the inner surface of the truncated cone. The frictional connection between said outer and inner surface may serve to prevent a not-intended relative movement of the light means inside the recess if the adjustment means are not moved by the passenger.

According to a third preferred embodiment the frame, in particular the ring-shaped element, has a spherical surface portion to pivot the ventilation nozzle having a corresponding inverted spherical surface portion. Preferably the centre point of said spherical surface portion and said ring-shaped element is identical. The corresponding inverted spherical portion of the ventilation nozzle may contact the spherical surface portion of the frame. The frictional connection between said surface portions prevents swivelling of the ventilation nozzle within the frame when no external force is applied. Alternatively, the ring shaped-element has an inner spherical surface portion to incorporate the ventilation nozzle.

According to another preferred embodiment the ring-shaped element comprises or consists of a transparent material. The transparent material may be glass or any kind of transparent plastics, so that a light beam of the light means can be transmitted. In this matter the openings of the recesses may be covered by transparent material. Alternatively, the ring-shaped element partly comprises transparent material, in particular for covering the openings of the recesses.

According to another preferred embodiment the adjustment means comprises a plurality of connecting elements having a first and a second end coupled at the first end to one of the light means and coupled at a second end to a holding element, and an adjustment pin coupled to the holding element. The connecting element, preferably a shaft, may have on said first end a form of a bracket to clamp the light means therein. The second end of the connecting element may have a ball to be inserted into a corresponding inverted spherical socket of the holding element. Accordingly, said second end of the connecting element and said spherical portion of the holding element may form a ball and socket joint. The holding element may be a ring having a linear extension on its outer circular edge. The ring may have a plurality of said spherical sockets. The number of spherical sockets may correspond to the number of connecting elements. The linear extension may have a cylindrical recess to incorporate the adjustment pin having a T-shape.

According to the previous embodiment the adjustment pin extends out of the surface of the PSU to shift the holding element, wherein the holding element is adapted to swivel each of the connecting elements, wherein each of the connecting elements is adapted to swivel the light means in relation to the ring-shaped element fixed to the frame. The pin may extend through a bore in the PSU. By pushing or swiveling the adjustment pin, the holding element may be shifted horizontally which effects swivelling of the connecting elements and the light means synchronously.

According to another preferred embodiment the light means comprises a light emitting diode (LED), preferably connected to a RGBW-controller. The RGBW controller may be connected to the LED by means of cables. Therefore, the RGBW controller is placeable variable. Each LED provides a light beam having a colour according to the characteristics of the LED. For example a LED comprising zinc selenide emits a blue light beam. The RGBW-controller provides mixing of the colours of various LEDs. Accordingly, the colour of the light beam, provided to the passenger, is variable and may be adapted to the time of the day or the interior design of the cabin.

According to another preferred embodiment an optical lens or a plurality of optical lenses is attached to or part of the light means. The optical lens which may partially have a spherical form and a U-form in cross-section, may be embedded into a plastic housing which is preferably screwed to the light means. A rigid connection between said light means and said optical lens effects a movement of the optical lens when the light means is swivelled. Alternatively, the adjustment means may be connected to the optical lenses only and swivel the optical lenses whereas the light generating element is fixed.

According to another preferred embodiment the optical lens(es) has/have an outer spherical surface for swivelling within the recess of the ring-shaped element. The outer spherical surface of the optical lens may contact the inner surface of the truncated cone of the recess in the ring-shaped element. Therefore, swivelling of the light means effects a relative movement of the optical lens to the ring-shaped element.

According to a further preferred embodiment the optical lens has a ring shape adapted to be attached to the plurality of light means. The optical lens may be a ring having a U-form in cross-section. The spherical surfaces of the LEDs may be glued to the inner surface of the optical lens, so that there is a rigid connection between said optical lens and the light means. Therefore, swivelling of the light means effects swivelling of the optical lens.

According to a further preferred embodiment the optical lens comprises at least one prism to deflect the light beam. The object of the prism is the deflection of the light beam emitted by the light means. If the light means with its optical lens is swivelled to an end position, a part of the light beam may be directed against the frame, so that the light beam projects only partly through the recess of the frame. Therefore, prisms are included in the optical lens to deflect the light beam through the recess of the frame.

According to a further preferred embodiment the holding element is attached to the PSU to produce a space between the ring-shaped element and the optical lens of each of the light means. The holding element may be pivotably mounted to the PSU or the frame. In this matter the light means do not contact the inner surface of the truncated cone of the recesses in the ring-shape element.

According to a final preferred embodiment a cooling channel is provided extending from the air nozzle to the light means. By this, a part of the ventilation air provided to the air nozzle is directed to the light means for cooling the light means. This will enhance life time of any light source of the light means and prevent the light means from being a hot spot adversely affecting the convenience of the passenger.

According to a further preferred embodiment, a cover element is provided said cover element having attachment means on a first face of said cover element for releasably mounting said cover element to said frame, and a design face on an opposite side of the cover element. Such a cover element can be used to adapt a PSU to specific design requirement or equipments inside an aircraft cabin. The design face of the cover element may be adapted in terms of design pattern and colour to a particular design pattern of an airline. Further, such cover element may be used to adapt a PSU to particular technical equipment which might be equipped to the PSU in later upgrade steps. To this regard, the cover element may comprise transparent sections allowing shining through of e.g. light of a light source which is mounted to said frame. Further, such transparent or semitransparent sections may have a specific form or contour effecting a signalling effect if light is emitted through said (semi-) transparent sections. This might be used to signalise fastening of the seat belt, non smoking or activated cabin attendant call through said cover element to the passenger.

A preferred embodiment of the invention is further explained with reference to the figures.
Figure 1 shows a cross-sectional view of a preferred embodiment of a PSU comprising a ventilation nozzle and a reading light.
Figure 2 shows a top view of the holding element.
Figure 3 shows partially the extension of the holding element.
Figure 4 shows the light means swivelled to its end position.

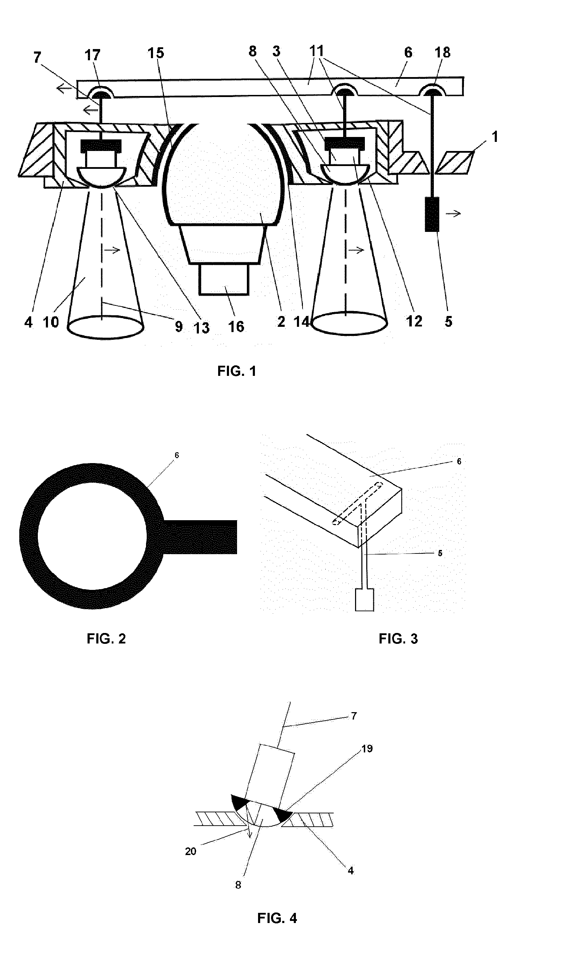

Referring first to figure 1, a frame (4) is glued to a passenger service unit (1). The frame (4) has a spherical surface portion (14) to pivot a ventilation nozzle (2) having a corresponding spherical surface portion (15). The ventilation nozzle (2) has an air outlet (16) which is faced towards the passenger providing an air flow.

Furthermore, a ring-shaped element (12) is integral in the frame (4) surrounding the ventilation nozzle (2) and comprises a plurality of recesses (13). Each of the recesses is a truncated cone to incorporate a light means (3) therein. The light means (3) comprises an optical lens (8), wherein the spherical portion of the optical lens contacts the recess (13) in the ring-shaped element (12).

For adjusting the projecting direction (9) of the light beam (10) an adjustment means (11) comprises an adjustment pin (5), a holding element (6) and a plurality of connecting elements (7). The connecting element (7) is a shaft having on its first end a form of a bracket to clamp the light means (3) therein. The second end of the connecting element (7) has a ball to be inserted into a corresponding inverted spherical socket (17) of the holding element (6). Therefore, the connection between said holding element (6) and the connecting elements (7) is a ball and socket joint. The holding element (6) is a ring having an extension on it outer circular edge. The extension has a cylindrical recess, wherein the adjustment pin (5) is swivel-mounted therein. The adjustment pin (5) extends through a bore in the PSU. The end of the adjustment pin (5) which extends out of the PSU facing towards the passenger, has a handle for grasping.

The arrows, illustrated in figure 1, show the mechanism when the adjustment pin (5) is actuated by the passenger. If the adjustment pin (5) is pushed in radial direction away from the ventilation nozzle (2) the holding element (6) is shifted in the opposite direction. Accordingly, the connecting elements (7) are either swivelled in the opposite direction of the adjustment pin (5), wherein the pivot point lies in the centre of the recess (13) of the ring-shaped element (12). The projecting direction (9) of the light beam (10) is swivelled in the identical direction of the adjustment pin (5), wherein the pivot point lies within the centre of the recess (13) of the ring-shaped element (12).

Referring to figure 2 the holding element (6) has a form of a ring having a linear extension which extends from an outer circular edge of the ring.

Referring to figure 3 the extension of the holding element (6) has a cylindrical recess (18). The adjustment pin (5) is movable embedded within said cylindrical recess (18).

Referring to figure 4 the light means is swivelled to its end position. Therefore, a part of the optical lens faces towards the upper surface of the frame, wherein the rays of light do partially not pass through the recess (13) of the ring-shaped element (12). The prisms (19), included in the optical lens, effect that the rays of light (20) are directed through the recess (13) of the ring-shaped element (12) towards the passenger.

## Claims

1. A passenger service unit comprising a ventilation nozzle and a reading light, with:
- a frame (1) connectable to a passenger service unit (2),
- at least one ventilation nozzle (3) mounted pivotably within the frame (1),
- a plurality of light means arranged circumferentially around the ventilation nozzle and projecting a light beam in a projecting direction, and
- an adjustment means adapted to adjust the projecting direction of the light beam,
**characterized in that**
the plurality of light means are mounted to a ring-shaped element fixed to the frame and the light means are moveable in relation to said ring-shaped element, wherein the adjustment means are coupled to the light means to effect a relative movement of the light means in relation to the ring-shaped element.

2. A passenger service unit according to claim 1,
wherein the ring-shaped element is integral with the frame surrounding the ventilation nozzle.

3. A passenger service unit according to claim 1,
wherein the ring-shaped element is a separate part attached immovable to the frame.

4. A passenger service unit according to the preceding claims,
wherein the ring-shaped element has a plurality of recesses incorporating the plurality of light means.

5. A passenger service unit according to claim 1 or 2,
wherein the frame, in particular the ring-shaped element, has an inner spherical surface portion to pivot the ventilation nozzle having a corresponding inverted spherical surface portion.

6. A passenger service unit according to any of the preceding claims,
wherein the ring-shaped element comprises or consists of a transparent material.

7. A passenger service unit according to any of the preceding claims,
wherein the adjustment means comprises a plurality of connecting elements with a first and a second end coupled end at the first end to one of the light means and coupled at the second end to a holding element, and
an adjustment pin coupled to the holding element.

8. A passenger service unit according to claim 7,
wherein the adjustment pin extends out of the surface of the passenger service unit and is adapted to shift the holding element,
wherein the holding element is adapted to swivel or move each of the connecting elements, and
wherein each of the connecting elements is adapted to swivel or move the light means in relation to the ring-shaped element fixed to the frame.

9. A passenger service unit according to any of the preceding claims,
wherein the light means comprises a light emitting diode, preferably connected to an RGBW-controller.

10. A passenger service unit according to any of the preceding claims,
wherein an optical lens or a plurality of optical lenses is attached to the light means.

11. A passenger service unit according to claim 10,
wherein the optical lens(es) has/have an outer spherical surface for swivelling within the recess of the ring-shaped element.

12. A passenger service unit according to claim 10,
wherein the optical lens has a ring shape adapted to be attached to the plurality of light means.

13. A passenger service unit according to any of the preceding claims,
wherein the optical lens comprises at least one prism to deflect the light beam.

14. A passenger service unit according to claim 7,
wherein the holding element is attached to the passenger service unit to produce a space between the ring-shaped element and the optical lens of each of the light means.
